# EUROPEAN PATENT SPECIFICATION

(11) **EP 1 758 616 B1**
(45) Date of publication and mention of the grant of the patent: **16.04.2008**
(21) Application number: 05751640.3
(22) Date of filing: 25.05.2005
(51) Int. Cl.: A61K 49/22, A61K 9/50

(54) **USE IN THE MEDICAL, DIAGNOSTIC AND PHLEBOLOGICAL FIELD OF A MIXTURE OF STERILE AND PHYSIOLOGICALLY ACCEPTABLE GASES**
VERWENDUNG EINES GEMISCHES AUS STERILEN UND PHYSIOLOGISCH ANNEHMBAREN GASEN AUF DEM MEDIZINISCHEN, DIAGNOSTISCHEN UND PHLEBOLOGISCHEN GEBIET
UTILISATION DANS LES DOMAINES MEDICAL, DIAGNOSTIQUE ET PHLEBOLOGIQUE D'UN MELANGE DE GAZ STERILES ET ACCEPTABLES D'UN POINT DE VUE PHYSIOLOGIQUE

(30) Priority: 26.05.2004 IT MI20041056
(43) Date of publication of application: 07.03.2007
(73) Proprietor: Tessari, Lorenzo, I-37019 Peschiera del Garda (IT); Cavezzi, Attilio, I-63039 S. Benedetto del Tronto (IT); Cabrera Garrido, Antonio Luis, 18005 Granada (ES)
(72) Inventor: Tessari, Lorenzo, I-37019 Peschiera del Garda (IT); Cavezzi, Attilio, I-63039 S. Benedetto del Tronto (IT); Cabrera Garrido, Antonio Luis, 18005 Granada (ES)
(74) Representative: Coppo, Alessandro
(86) International application number: PCT/IB2005/001693
(87) International publication number: WO 2005/115484

(56) References cited:
- EP-A- 0 586 875
- EP-A- 0 656 203
- WO-A-96/38180
- US-A- 5 556 610
- US-A1- 2002 077 589
- PORTER T R ET AL: "INTERACTION OF DIAGNOSTIC ULTRASOUND WITH SYNTHETIC OLIGONUCLEOTIDE-LABELED PERFLUOROCARBON-EXPOSED SONICATED DEXTROSE ALBUMIN MICROBUBBLES" JOURNAL OF ULTRASOUND IN MEDICINE, SAUNDERS, PHILADELPHIA, PA, US, vol. 15, no. 8, August 1996 (1996-08), pages 577-584, XP002054859 ISSN: 0278-4297
- FORSBERG F ET AL: "Effect of filling gases on the backscatter from contrast microbubbles: theory and in vivo measurements" ULTRASOUND IN MEDICINE AND BIOLOGY, NEW YORK, NY, US, vol. 25, no. 8, October 1999 (1999-10), pages 1203-1211, XP004295454 ISSN: 0301-5629

## Description

The present invention relates to the use of a mixture of sterile and physiologically acceptable gases in the medical, phlebological and diagnostic field.

The present invention originates and can be applied in the diagnostic field, as a contrast means for echography, and in the medical field in phlebology and medical therapy in general.

In particular, the gas mix of the invention allows stable and physiologically acceptable sterile microbubbles to be produced, by means of a standardized and simple procedure.

The systemic use of contrast means for ultrasounds or ultrasoundgraphs (MCU) has been known for quite a time in the diagnostic field. A typical example of contrast means consists of microbubbles containing a bio-compatible gas. Once these microbubbles have been introduced into the human body, they cause a variation in the tissue characteristics of reflectivity, absorption and passage rate of the ultrasound beam. The main function of these contrast means is to increase the reflectivity of a tissue or organ with respect to the surrounding area.

This effect can be obtained by increasing the reflectivity, or rather the signal/sound ratio, thanks to the introduction of microbubbles into the bloodstream.

As microbubbles consisting of gases have an echo-genicity coefficient higher than the solid and liquid echografic means, when the surrounding medium is a liquid, as in the case of the bloodstream, the use of gases as a contrast medium in echography, echocardiography, vascular echocolordoppler, etc.., has become increasingly more widespread.

When microbubbles based on gas are subjected to the action of ultrasounds, they cause a satisfactory refraction-reflection process so as to reveal, with echocolordoppler or echography, the relevant images as hyper-echogenic or chromatically typical.

Notoriously, the contrast means for ultrasounds based on gases must satisfy various conditions.

Firstly, they must be physiologically acceptable and have a sufficiently long persistence in the bloodstream to allow the necessary evaluations to be effected, for obtaining an adequate diagnostic response.

Furthermore, the microbubbles must have a sufficiently reduced dimension; this requirement jeopardizes the persistence, as it has been verified that once microbubbles having a diameter lower than 10 microns have been introduced into the circulatory flow, they only survive for 0.25 sec.

Gas having a high molecular weight was therefore used to produce bubbles, in an attempt to overcome this limit. The use of air and gases having a high molecular weight, such as C₂H₆, SF₆, C₄F₈ was also proposed for producing microbubbles.

It has been found however that the systemic administration of microbubbles containing said gases for echo-genic purposes, can cause side-effects in the circulatory system, above all when the investigation is laborious and requires the administration of relevant amounts of gas.

Also in the phlebological field, where air has been widely used as a carrier medium of sclerosing substances, cases of transitory neurological deficits have been reported, as well as doubtful pulmonary embolisms; this makes it necessary to use reduced amounts of sclerosing foam and consequently of air.

In order to avoid the occurrence of these side-effects, resort was made to CO₂, a highly bio-compatible gas, as a carrier medium for sclerosing substances.

It was found however that although the use of CO₂ was able to cause a reduction in the appearance of side-effects in the circulatory system, it is not satisfactory with respect to the persistence of the microbubbles inside the syringe, before the injection of the sclerosing foam, and bloodstream. Once microbubbles containing carbon dioxide have been administered, they do in fact tend to explode or blend together forming bubbles with larger dimensions considered unsuitable for the purposes of sclerosing treatment. This is due to the greater diffusibility of CO₂ with respect to other gases. EP 0 656 203 A discloses an injectable micro-foam for sclerotherapy comprising a gas mixture oxygen and carbon dioxide. US 5 556 610 A discloses injectable media for ultra sonic echography in the form of microbubbles comprising air as a gas component.

The necessity is therefore currently felt for availing of methods for the production of biocompatible microbubbles which can be used in the medical, diagnostic and phlebological field.

One of the main objectives of the present invention consists in providing a mixture of physiologically acceptable gases, which can be used in a more or less standardized manner, for producing microbubbles suitable as carriers of sclerosing agents, or other drugs.

Another objective of the invention consists in providing a mixture of biocompatible sterile gases capable of forming microbubbles which are well tolerated and having persistence in the bloodstream suitable for effecting the evaluations necessary for obtaining an adequate diagnostic response.

A last objective is to provide a method for the extemporaneous formation of microbubbles whose systemic administration for diagnostic or therapeutic use is practically exempt from causing strong side-effects due to the gaseous component of the gas mix and active principle.

The applicants have found that it is possible to produce stabile microbubbles with safe medical use, by adopting a gaseous mixture based on two selected physiologically acceptable gases.

In view of the objectives indicated above and others which will appear more evident in the following description, a first aspect of the invention relates to the use of a composition of sterile gases, soluble at atmospheric pressure and physiologically acceptable for the production of microbubbles as a contrast means for ultrasounds, wherein said composition comprises oxygen and carbon dioxide wherein said mixture comprises CO₂ in a quantity ranging from 50 to 95% by volume with respect to the total volume of gas in the mixture and optionally at least another gas in a quantity not higher than 10% by volume with respect to the total volume of said mixture.

According to another aspect of the present invention, the use is provided of a composition of sterile gases, soluble at atmospheric pressure and physiologically acceptable for the production of microbubbles for sclerotherapy, wherein said composition comprises oxygen and carbon dioxide wherein said mixture comprises CO₂ in a quantity ranging from 50 to 95% by volume with respect to the total volume of gas in the mixture and optionally at least another gas in a quantity not higher than 10% by volume with respect to the total volume of said mixture.

The applicants have found that the use of oxygen and carbon dioxide, combined with each other, unexpectedly produce a synergic effect on the stabilization of the microbubbles formed, causing an increase in their persistence in the bloodstream which, in echographic diagnostics, allows the necessary evaluations to be effected for obtaining an adequate response and, in phlebological therapy, an adequate sclerosing treatment.

The mixture of O₂ and CO₂ is capable of effectively counterbalancing the surface tension of the microbubbles which is caused when they are inside the circulatory stream. Furthermore, the stabilization which is obtained with the combined use of CO₂ and O₂ is temporary, which allows an easy elimination of the microbubbles on the part of the organism thus avoiding any type of toxicity for the patient. When the microbubbles, in fact, collapse inside the blood veins, they release two non-toxic gases as they are physiologically already present in the organism.

In particular, the authors have found that a mixture of gases essentially based on O₂ and CO₂ has a higher density, a lower diffusibility and a lower saturation constant with respect to the air. The combination of these characteristics significantly contributes to optimizing the life duration of the microbubbles once they have been injected into the bloodstream.

Within the scope of the invention, the specific characteristics of CO₂ such as maximum tolerability, strong tendency to bind itself to the vein wall carrying the sclerosing drug, are synergically combined with those of oxygen obtaining a stabilization of the microbubbles which balances the greater tendency to coalescence that the same microbubbles would have if their single gas consisted of CO₂ alone.

Within the scope of the present invention, the term microbubbles is meant to comprise bubbles having a diameter ranging from 5 to 200 and preferably from 10 to 50 microns.

The formation of microbubbles with these dimensions minimizes the risks of embolism and the creation of side-effects which are dangerous for the patient's health.

In the mixture of the invention, the volume ratios between O₂ and CO₂ can typically vary within a wide range, for example from 5% to 95% by volume. It has been found, however, that when CO₂ is present within a range of 50 to 95% by volume and preferably 60-80% by volume, the mixture proves to be highly appropriate for the formation of particularly stable and biocompatible microbubbles.

Other gases are advantageously not present in the mixture of the invention, or should they be so, these additional gases are present in a quantity which does not exceed 10% by volume with respect to the final gaseous mix.

According to an embodiment, the composition of the invention can also comprise one or more biologically acceptable gases such as, for example, air, nitrogen, ozone. As previously specified, these gases, when present, do not exceed 10% by volume and are preferably present in a quantity ranging from 1-5% with respect to the total volume of gases present in the mixture.

The mixture of gases used for producing the microbubbles is apyrogenic and sterilized before use or packaging, for example by exposure to gamma rays or to ethylene oxide or other known sterilization forms.

In diagnostic use, the composition based on a mixture of O₂ and CO₂ according to the invention is systemically administered as such or combined with foaming carrying means. The administration is typically effected by intravenous injection.

Foaming carrying means which can be used are physiologically acceptable means which are appropriate contrast means for echography, once introduced into the circulatory stream. A typical carrying means comprises albumin but also polysorbate, in its various chemical forms.

For the specific uses of the invention, the composition is conveniently packaged and preserved inside a suitable container, typically consisting of a glass bottle for medical use.

The characteristics and advantages of the use of a mixture of oxygen and carbon dioxide, according to the present invention will appear more evident from the following illustrative and non-limiting description, referring to the enclosed schematic drawings, in which:
figure 1 shows a raised side view of a container for a composition of sterile gases of the invention;
figure 2 is a plan view from above of a container for a composition of sterile gases of the invention;
figure 3 illustrates a plan view from below of the container of the previous figures and
figure 4 shows a perspective of the container of the previous figures.

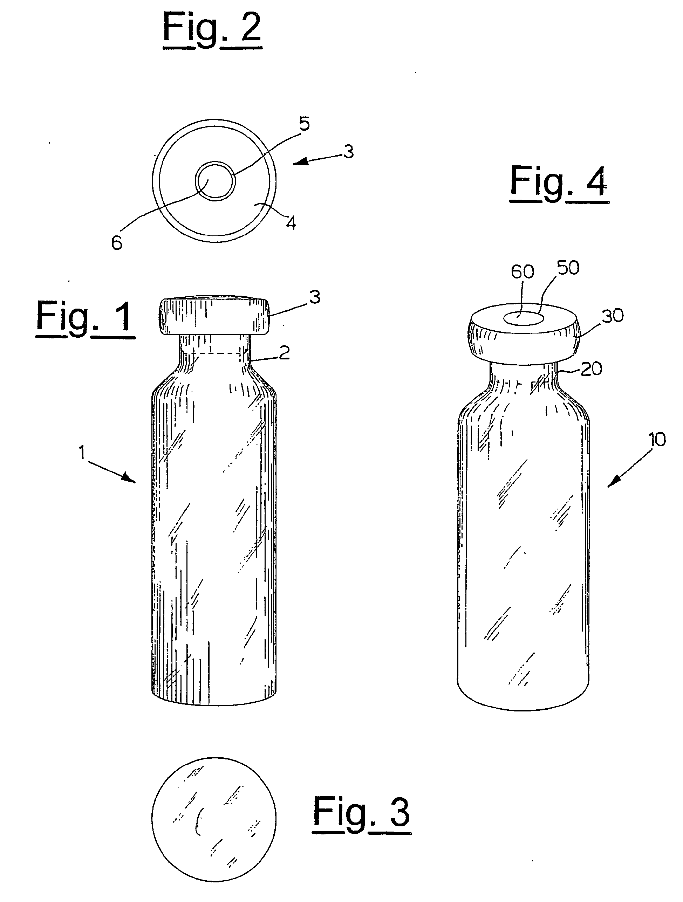

With reference to figure 1, this shows a container 1 for a composition of sterile gases of the invention, typically consisting of a glass bottle with a constriction or neck 2 on whose upper end a ring 3 typically made of metal, is attached. As can be observed from figure 2, said ring 3 has a covering portion 4 having a central hole 5. The metal ring 3 constrains a washer 6 to the upper end of the neck 2 of the bottle creating a tight seal and preventing the mixture of oxygen and gas contained in the container 1 from leaking.

Figure 3 indicates that the lower end of the container 1 is closed and is made of the same material as the container. The washer is advantageously capable of offering a seal at an internal pressure ranging from 1.1 to 5 atmospheres. For this purpose, the ring 30 can be made of aluminum with a typical thickness varying from 0.1 to 2 mm. The bottle conveniently has a capacity varying from 5-10 cc approximately.

With reference to figure 4, in order to administer the composition of the invention to a patient for diagnostic purposes, it is sufficient to remove, for example with a syringe, a suitable quantity of mixture of O₂ and CO₂ by perforating the washer 60 of the container 10 with a needle and subsequently administering the gas mix, intravenously.

Analogously, it is possible to produce microbubbles suitable for therapeutic purposes by removing the gas mix from the bottle 10 with a syringe and extemporaneously preparing a sclerosing foam according to one of the techniques known to experts in the field which envisage the turbulent mixing of gas and a liquid.

According to an alternative embodiment, the bottle 10 is equipped with a filling and removal system of a fluid provided with valve means. More specifically, these valve means comprise a mobile shutting element between a position which allows the extraction or release of the fluid and a closed position. For this purpose, the perforating element has specific elastic means suitable for keeping the perforating element itself in a closed position.

It is possible for example to produce a sclerosing foam by resorting to: a) the method described by Tessari in US patent application 2002/0077589 A1, b) the method described by A.L. Cabrera Garrido in Spanish patent application Nr. P 200301995 or alternatively, c) with the device for the production of a foam for diagnostic or therapeutic use described by Attilio Cavezzi and Lorenzo Tessari in Italian patent application MI 2003 #1204.

Once the sclerosing foam obtained has been injected into the vessel to be treated, thanks to the presence of oxygen, it proves to be capable of persisting for a sufficient time for allowing the action of the sclerosing agent on the innermost walls of the vessels. Furthermore, the corresponding presence of CO₂, which tends to permeate blood vessels more rapidly, allows a uniform distribution of the foam on the inner surfaces of the vessels.

The packaging in bottles having varying dimensions of gas mixtures for the uses of the invention enables the preparation procedure of microbubbles/foams for sclerosing therapy to be further standardized and simplified.

According to a preferred embodiment, in order to form a foam with microbubbles, the mixture containing O₂/CO₂ is mixed with a liquid or mixture of foaming liquids suitable for therapeutic or diagnostic use. The neo-formed foam is revealed by echographic/ultrasoundgraphic means currently available on the market.

In particular, the formation of foam is obtained by mixing, under turbulence conditions, a physiologically acceptable foaming liquid with the gaseous mixture based on CO₂/O₂ of the invention.

For the formation of a foam, a turbulence is required which can be created, for example, by passing the liquid and the gas mixture through a constriction/passage with a brusque enlargement. Optionally, it is possible to add a foaming substance such as a surface-active agent in order to improve the dispersion of gas bubbles.

When the method according to Tessari described for example in GB patent application 2 369 996 A is used, it is the passage of the sclerosing-gas liquid mixture inside a three-way tap which creates the turbulence and final foam. When, on the other hand, the method described by Antonio Cabrera Garrido, for example in patent Nr. P 200301995 is used, the disposable syringe contains the suitable quantity of sclerosing liquid and gas soluble in the blood at atmospheric pressure; the syringe is then shaken. The shaking of the syringe and its contents causes a dispersion with the liquid as a continuous system and the gas as a discontinuous system i.e. microbubbles.

The foaming liquid is conveniently a surface-active sclerosing drug for therapeutic use capable of forming a foam and sclerosing venous vessels, when injected into the lumen of the vessel.

Examples of suitable sclerosing substances comprise surface-active agents such as sodium tetradecyl sulfate, polydocanol, ethanolamine oleate, sodium morruate, or other sclerosing agents.

According to another embodiment, said liquid for diagnostic use is a biological foaming liquid for diagnostic use i.e. capable of forming a foam with a physiologically acceptable gas which can be revealed by means of an echographic/ultrasoundgraphic apparatus.

Biological foaming liquids comprise albumin, plasma, or inert and physiologically acceptable foaming substances.

According to another aspect of the present invention, a method is therefore provided for the production of a foam suitable for diagnostic or therapeutic application comprising the transfer of the mixture of CO₂ and O₂ of the invention and a pharmaceutically acceptable foaming liquid through one or more constrictions for mixing the gas and liquid under turbulence conditions and forming a foam and maintaining the foam under pressure until the moment of use.

The ratio of the gas mixture with respect to the liquid is advantageously pre-established so as to produce a foam in which the gaseous microbubbles are finely dispersed, creating a density conveniently ranging from 0.09 to 0.40 g/ml.

The ratio of the volume of the gas mixture with respect to the liquid preferably ranges from 2:1 to 10:1, more preferably from 3:1 to 7:1, and even more preferably is equal to 4:1 with the Tessari method described for example in GB patent application 2 369 996 A.

With the typical syringe shaker of the Antonio Cabrera method, the shaking time typically ranges from 30 seconds to 5 minutes. The liquid/gas ratio preferably ranges from 1 to 4. The dimension of the microbubbles is typically equal to about 50 microns, the density of the foam depends on the concentration of surface-active agent and varies from 0.2 to 4 g per cm³. In the case of the use of a sclerosing substance as a therapeutic foaming liquid, the concentration of the sclerosing agent is conveniently within the range of concentrations commonly used in sclerotherapy. Furthermore, in the case of a mixture with other types of drugs, for example local anaesthetics, anti-inflammatory substances etc., foaming agents in themselves or made to be as such, the foam obtained with the gas mix herein disclosed, can be simply and extemporaneously produced with complete safety and sterility; the parenteral injection of said drugs in extravasal foam form, can in fact have advantages with respect to liquid or solid form such as a greater persistence in loco, a longer duration and, hypothetically, greater strength. The gas mixture of the invention is therefore useful in extravasal parenteral use due to the facility and innocuity of the reabsorption in the tissues of its two components (O₂ and CO₂).

The fact that the gas mixture of the invention allows a foam for therapeutic or diagnostic use to be extemporaneously produced and with greater standardization, is also particularly advantageous.

The possibility of using pre-established quantities of gas mix and therapeutic substance or diagnostic agent, adopting a foam production method which is also highly standardized, provides doctors with a procedure which is simple to put into practice and which also has a high safety profile.

In particular, a foam rich in microbubbles can be formed by sending a pre-established gas/liquid foaming mixture, for a preselected number of times inside the mixing device, for example a syringe which allows varying pressure values to be exerted on the gas/liquid mixture, by means of piston-like operation.

The following examples are provided for purely illustrative purposes of the present invention and should in no way be considered as limiting its protection scope as defined in the enclosed claims.

### EXAMPLE 1

A mixture containing CO₂ 60% by volume and O₂ 40% by volume is packaged in a 5 cc bottle at a pressure of 3 atmospheres. Once the gas mixture has been sterilized by means of gamma rays, it is ready for the extemporaneous formation of microbubbles.

### EXAMPLE 2

Microbubbles were produced, having a diameter of 6 microns by removing with a 5 cc syringe, a mixture of 65% of CO₂, 38% of O₂ and 2% of ozone contained in a sterile bottle. The gas mixture was mixed with sodium tetradecyl sulfate and/or polydocanol according to the method described by Tessari or Cabrera with the formation of a foam consisting of microbubbles which were injected into a vein to be sclerosed.

## Claims

1. Use of a composition of sterile gases, soluble at atmospheric pressure and physiologically acceptable for the production of microbubbles as contrast means for ultrasounds, wherein said composition comprises oxygen and carbon dioxide wherein said mixture comprises CO₂ in a quantity ranging from 50 to 95% by volume with respect to the total volume of gas in the mixture and optionally at least another gas in a quantity not higher than 10% by volume with respect to the total volume of said mixture.

2. Use of a composition of sterile gases, soluble at atmospheric pressure and physiologically acceptable for the production of microbubbles for sclerotherapy, wherein said composition comprises oxygen and carbon dioxide wherein said mixture comprises CO₂ in a quantity ranging from 50 to 95% by volume with respect to the total volume of gas in the mixture and optionally at least another gas in a quantity not higher than 10% by volume with respect to the total volume of said mixture.

3. Use of a composition of sterile gases, soluble at atmospheric pressure and physiologically acceptable for the production of microbubbles for carrying at least one drug in the form of a foam for parenteral use, wherein said composition comprises oxygen and carbon dioxide wherein said mixture comprises CO₂ in a quantity ranging from 50 to 95% by volume with respect to the total volume of gas in the mixture and optionally at least another gas in a quantity not higher than 10% by volume with respect to the total volume of said mixture.

4. Use according to anyone of claims 1-3, wherein the CO2 is present in a quantity ranging from 60 to 80% by volume with respect to the total volume of gas in the mixture.

5. Use according to any of the claims 1-4, wherein said other gas is selected from ozone, air, nitrogen, and mixtures thereof.

6. Use according to any of the claims 1-4 wherein said gas mixture essentially consists of O2 and CO2.

7. Use according to any of the claims 1-6, wherein the microbubbles produced have a diameter ranging from 10 to 50 microns.

8. Use according to any of the claims 1-7, wherein said composition of sterile gases is packaged in a sterile disposable container.

9. Use according to claim 1, wherein said microbubbles are produced by mixing said composition of sterile gases, under turbulence, with a physiologically acceptable foaming liquid.

10. Use according to claim 9, wherein said foaming liquid is selected from albumin, plasma, polysorbates and mixtures thereof.

11. Use according to claim 2, wherein said microbubbles are produced by mixing said composition of sterile gases, under turbulence, with a sclerosing substance.

12. Use according to claim 11, wherein said sclerosing substance is a sclerosing surface-active agent, or a sclerosing drug transferred into a foam by means of foaming additives.

13. Use according to claim 12, wherein said sclerosing substance is selected from sodium tetradecyl sulfate, polydocanol, ethanolamine oleate, sodium morruate, poly-docanol and mixtures thereof.

## Patentansprüche

1. Verwendung einer Zusammensetzung von sterilen Gasen, die bei atmosphärischem Druck löslich sind und die physiologisch für die Produktion von Mikrobläschen als Kontrastmittel für Ultraschall akzeptabel sind, wobei die Zusammensetzung Sauerstoff und Kohlendioxid beinhaltet, wobei die Mischung CO₂ in einer Menge beinhaltet, die mit Bezug auf das Gesamtvolumen von Gas in der Mischung von 50 bis 95 Vol.-% reicht, und optional mindestens ein anderes Gas in einer Menge, die mit Bezug auf das Gesamtvolumen der Mischung nicht größer als 10 Vol.-% ist, beinhaltet.

2. Verwendung einer Zusammensetzung von sterilen Gasen, die bei atmosphärischem Druck löslich sind und die physiologisch für die Produktion von Mikrobläschen zur Sklerotherapie akzeptabel sind, wobei die Zusammensetzung Sauerstoff und Kohlendioxid beinhaltet, wobei die Mischung CO₂ in einer Menge beinhaltet, die mit Bezug auf das Gesamtvolumen von Gas in der Mischung von 50 bis 95 Vol.-% reicht, und optional mindestens ein anderes Gas in einer Menge, die mit Bezug auf das Gesamtvolumen der Mischung nicht größer als 10 Vol.-% ist, beinhaltet.

3. Verwendung einer Zusammensetzung von sterilen Gasen, die bei atmosphärischem Druck löslich sind und die physiologisch für die Produktion von Mikrobläschen zum Tragen von mindestens einem Arzneimittel in der Form eines Schaums zur parenteralen Verwendung akzeptabel sind, wobei die Zusammensetzung Sauerstoff und Kohlendioxid beinhaltet, wobei die Mischung CO₂ in einer Menge beinhaltet, die mit Bezug auf das Gesamtvolumen von Gas in der Mischung von 50 bis 95 Vol.-% reicht, und optional mindestens ein anderes Gas in einer Menge, die mit Bezug auf das Gesamtvolumen der Mischung nicht größer als 10 Vol.-% ist, beinhaltet.

4. Verwendung gemäß einem der Ansprüche 1-3, wobei das CO₂ in einer Menge vorhanden ist, die von 60 bis 80 Vol.-% mit Bezug auf das Gesamtvolumen von Gas in der Mischung reicht.

5. Verwendung gemäß einem der Ansprüche 1-4, wobei das andere Gas aus Ozon, Luft, Stickstoff und Mischungen davon ausgewählt ist.

6. Verwendung gemäß einem der Ansprüche 1-4, wobei die Gasmischung im Wesentlichen aus O₂ und CO₂ besteht.

7. Verwendung gemäß einem der Ansprüche 1-6, wobei die produzierten Mikrobläschen einen Durchmesser aufweisen, der von 10 bis 50 Mikrometer reicht.

8. Verwendung gemäß einem der Ansprüche 1-7, wobei die Zusammensetzung von sterilen Gasen in einem sterilen Einweg-Behälter verpackt ist.

9. Verwendung gemäß Anspruch 1, wobei die Mikrobläschen durch das Mischen der Zusammensetzung von sterilen Gasen unter Turbulenz mit einer physiologisch akzeptablen schäumenden Flüssigkeit produziert werden.

10. Verwendung gemäß Anspruch 9, wobei die schäumende Flüssigkeit aus Albumin, Plasma, Polysorbaten und Mischungen davon ausgewählt ist.

11. Verwendung gemäß Anspruch 2, wobei die Mikrobläschen durch das Mischen der Zusammensetzung von sterilen Gasen unter Turbulenz mit einer sklerosierenden Substanz produziert werden.

12. Verwendung gemäß Anspruch 11, wobei die sklerosierende Substanz ein sklerosierendes oberflächenaktives Mittel oder ein sklerosierendes Arzneimittel ist, das durch schäumende Zusatzstoffe in einen Schaum übertragen wird.

13. Verwendung gemäß Anspruch 12, wobei die sklerosierende Substanz aus Natriumtetradecylsulfat, Polydocanol, Ethanolaminoleat, Natriummorrhuat, Poly-Docanol und Mischungen davon ausgewählt ist.

## Revendications

1. Utilisation d'une composition de gaz stériles, solubles à la pression atmosphérique et acceptables d'un point de vue physiologique pour la production de microbulles comme moyen de contraste pour des ultrasons, dans laquelle ladite composition comprend de l'oxygène et du dioxyde de carbone où ledit mélange comprend du CO₂ dans une quantité comprise dans la gamme allant de 50 à 95 % en volume par rapport au volume total de gaz dans le mélange et de façon facultative au moins un autre gaz dans une quantité n'excédant pas 10 % en volume par rapport au volume total dudit mélange.

2. Utilisation d'une composition de gaz stériles, solubles à la pression atmosphérique et acceptables d'un point de vue physiologique pour la production de microbulles pour la sclérothérapie, dans laquelle ladite composition comprend de l'oxygène et du dioxyde de carbone où ledit mélange comprend du CO₂ dans une quantité comprise dans la gamme allant de 50 à 95 % en volume par rapport au volume total de gaz dans le mélange et de façon facultative au moins un autre gaz dans une quantité n'excédant pas 10 % en volume par rapport au volume total dudit mélange.

3. Utilisation d'une composition de gaz stériles, solubles à la pression atmosphérique et acceptables d'un point de vue physiologique pour la production de microbulles destinées à porter au moins un médicament sous la forme d'une mousse pour usage parentéral, dans laquelle ladite composition comprend de l'oxygène et du dioxyde de carbone où ledit mélange comprend du CO₂ dans une quantité comprise dans la gamme allant de 50 à 95 % en volume par rapport au volume total de gaz dans le mélange et de façon facultative au moins un autre gaz dans une quantité n'excédant pas 10 % en volume par rapport au volume total dudit mélange.

4. Utilisation selon n'importe laquelle des revendications 1 à 3, dans laquelle le CO₂ est présent dans une quantité comprise dans la gamme allant de 60 à 80 % en volume par rapport au volume total de gaz dans le mélange.

5. Utilisation selon n'importe lesquelles des revendications 1 à 4, dans laquelle ledit autre gaz est sélectionné parmi l'ozone, l'air, l'azote, et des mélanges de ceux-ci.

6. Utilisation selon n'importe lesquelles des revendications 1 à 4, dans laquelle ledit mélange de gaz consiste essentiellement en O₂ et CO₂.

7. Utilisation selon n'importe lesquelles des revendications 1 à 6, dans laquelle les microbulles produites ont un diamètre compris dans la gamme allant de 10 à 50 microns.

8. Utilisation selon n'importe lesquelles des revendications 1 à 7, dans laquelle ladite composition de gaz stériles est conditionnée dans un récipient jetable stérile.

9. Utilisation selon la revendication 1, dans laquelle lesdites microbulles sont produites en mélangeant ladite composition de gaz stériles, sous turbulence, avec un liquide moussant acceptable d'un point de vue physiologique.

10. Utilisation selon la revendication 9, dans laquelle ledit liquide moussant est sélectionné parmi l'albumine, le plasma, les polysorbates et des mélanges de ceux-ci.

11. Utilisation selon la revendication 2, dans laquelle lesdites microbulles sont produites en mélangeant ladite composition de gaz stériles, sous turbulence, avec une substance sclérosante.

12. Utilisation selon la revendication 11, dans laquelle ladite substance sclérosante est un agent tensio-actif sclérosant, ou un médicament sclérosant transféré dans une mousse au moyen d'additifs moussants.

13. Utilisation selon la revendication 12, dans laquelle ladite substance sclérosante est sélectionnée parmi le tétradécyl sulfate de sodium, le polidocanol, l'oléate d'éthanolamine, le morrhuate de sodium, le poli-docanol et des mélanges de ceux-ci.
